# EUROPEAN PATENT SPECIFICATION

(11) **EP 0 522 677 B1**
(45) Date of publication and mention of the grant of the patent: **18.12.1996**
(21) Application number: 92302080.4
(22) Date of filing: 11.03.1992
(51) Int. Cl.: G01N 21/76, G01N 33/53, G01N 33/533

(54) **Method of measuring concentration of immunoreactant using electrochemiluminescence**
Verfahren zur Messung der Konzentration eines Immuno-Reaktanden unter Verwendung von Elektrochemilumineszenz
Procédé pour le mesurage de la concentration d'immunoréactant au moyen luminescence électrochimique

(30) Priority: 10.07.1991 JP 195807/91; 10.07.1991 JP 195808/91
(43) Date of publication of application: 13.01.1993
(73) Proprietor: TDK Corporation, Chuo-ku, Tokyo-to 103 (JP)
(72) Inventor: Shibue, Akira, Koganei-shi, Tokyo (JP); Tanaka, Masaru, Chiba-shi, Chiba-ken (JP); Kamiya, Shinji, Koganei-shi, Tokyo (JP)
(74) Representative: Votier, Sidney David

(56) References cited:
- EP-A- 0 273 115
- EP-A- 0 330 050
- EP-A- 0 361 817
- WO-A-89/10552
- WO-A-90/11511
- GB-A- 1 316 363
- GB-A- 1 461 877
- GB-A- 2 233 450
- US-A- 3 352 791
- US-A- 4 946 958

## Description

### BACKGROUND OF THE INVENTION

### 1. Field of the Invention

The present invention relates to a method of measuring the concentration of an immunoreactant present in a liquid by utilizing electrochemiluminescence.

### 2. Description of the Prior Art

Methods of measuring the concentration of an immunoreactant, such as antigen or antibody, present in a liquid by utilizing electrochemiluminescence have been known. For example, a method has been known in which an antigen complementary to an antibody contained in a liquid sample is preliminarily labeled with a chemiluminescent substance such as luminol, pyrene, etc. The labeled antigen is added to the liquid sample to react with the antibody, whereby chemiluminescence based on electrochemical reaction (electrochemiluminescence) of the chemiluminescent substance attached to the antigen as a marker is suppressed due to the immunoreaction. Utilizing the suppression, the concentration of the antibody in the liquid sample is determined by measurement of the amount of electrochemiluminescence.

The method, however, has the drawback that change in the amount of luminescence due to change in concentration of antigen or antibody is little and, therefore, accuracy of measurement is law.

Measurement of electrochemiluminescent phenomena is e.g. described in WO 89/10552.

### SUMMARY OF THE INVENTION

Accordingly it is an object of the present invention to provide a method of measuring the concentration of an immunoreactant which gives a large change in the amount of luminescence due to change in concentration of the immunoreactant to be measured and, therefore, ensures an improved accuracy of measurement.

The above object is attained according to the present invention by preparing an electrode with an immunoreactant immobilized thereon, binding an immunoreactant labeled with a chemiluminescent acridinium compound to the electrode through an immunoreaction, or antigen-antibody reaction, in a liquid sample, then performing electrochemical reduction with the electrode as a working electrode to thereby trigger chemiluminescence, and measuring the amount of luminescence.

According to the present invention, there is provided a method of measuring the concentration of an immunoreactant in a liquid sample, comprising the steps of:
adding to the immunoreactant-containing liquid sample an immunoreactant competitive with the immunoreactant in the liquid sample, said competitive immunoreactant having been previously labeled with a chemiluminescent acridinium compound;
causing an immunoreaction between the unlabeled and labeled immunoreactants with a complementary immunoreactant which is capable of specifically combining with the immunoreactants and is immobilized on an electrode, to allow competitive binding of the unlabeled and labeled immunoreactants onto the electrode to take place;
washing the electrode to remove the excess or unbound unlabeled and labeled immunoreactants; and
subjecting dissolved oxygen in an electrolytic solution to electrochemical reduction using the washed electrode as a working electrode to generate luminescence; and
measuring the amount of luminescence. (This method will be hereinafter referred to as "the first invention".)

According to the present invention, there is also provided a method of measuring the concentration of an immunoreactant in a liquid sample, comprising the steps of:
causing an immunoreaction between the immunoreactant with a complementary immunoreactant which is capable of specifically combining with the immunoreactant to be measured and is immobilized on an electrode in the liquid sample, to allow binding of the immunoreactant in the liquid sample onto the electrode to take place;
adding an excess of a complementary immunoreactant labeled with a chemiluminescent acridinium compound to the liquid thus obtained, to allow the bound immunoreactants to react with the labeled complementary immunoreactant;
washing the electrode to remove the excess or unbound labeled complementary immunoreactant; and
subjecting dissolved oxygen in an electrolytic solution to electrochemical reduction using the washed electrode as a working electrode to generate luminescence; and
measuring the amount of luminescence. (This method will be hereinafter referred to as "the second invention".)

The methods according to the present invention are effective for measurement of antigen or antibody concentrations ranging from about 10⁻¹¹ to about 10⁻⁵ g/ml. The methods of the present invention give a large change in the amount of luminescence due to change in the antigen or antibody concentration to be measured, and offer markedly improved accuracy of measurement, as compared to the prior art methods.

### BRIEF DESCRIPTION OF THE DRAWINGS

Figure 1 is a schematic illustration of the steps of the first invention;
Figure 2 is a diagram showing the pattern of a calibration curve used for calculating the antigen content in a liquid sample from the amount of electrochemiluminescence, in carrying out the method according to the first invention;
Figure 3 is a schematic illustration of the steps of the second invention;
Figure 4 is a diagram showing the pattern of a calibration curve used to calculate the antigen content in a liquid sample from the amount of electrochemiluminescence, in carrying out the method according to the second invention;
Figure 5 is a diagram showing the measurement results in Example 1 which is a working example of the first invention;
Figures 6 and 7 are diagrams showing the results of measurements in Examples 2 and 3, respectively which are working examples of the second invention.

### DETAILED DESCRIPTION OF THE INVENTION

In the present invention, the immunoreactant means an antigen or antibody, and the complementary immunoreactant means an antibody or antigen capable of specifically binding to said antigen or antibody. Typical but not limitative examples of the immunoreactant and the complementary immunoreactant include the followings:
- Antigens: IgG, IgA, IgM, IgE, albumin, HCG, AFP, cardinolipin antigen, blood group substances, concanavalin A, DNT, prostaglandin, CRP, HBs, human growth hormones, steroid hormones,CEA, IgD, etc.
- Antibodies: anti-albumin antibody, anti-HCG antibody, anti-IgG antibody, anti-IgA antibody, anti-IgM antibody, anti-IgE antibody, anti-IgD antibody, anti-AFP antibody, anti-DNT antibody, anti-prostaglandin antibody, anti-human-coagulation-factor antibody, anti-CRP antibody, anti-HBs antibody, anti-human-growth-hormone antibodies, anti-steroid hormone antibodies, sera containing such antibodies as above, and monoclonal antibodies.

### Electrode

In the methods according to the present invention, use is made of an electrode on which a complementary immunoreactant capable of specifically combining with an immunoreactant to be measured is immobilized. Immobilization of the complementary immunoreactant onto the electrode can be achieved by conventional methods, for instance, by coating the electrode with a silane coupling agent or with protein A obtained from staphylococcus and then binding the complementary immunoreactant to the coating on the electrode.

The electrode on which the complementary immunoreactant is immobilized may be any electrode, provided that effective immobilization is possible. In general, however, metal electrodes such as platinum electrodes, gold electrodes, etc., carbon electrode such as graphite, glassy carbon, etc., and oxide electrodes such as ITO electrodes, etc. are used preferably.

### Chemiluminescent substance

In the method according to the present invention, a chemiluminescent acridinium compound is used for labeling an immunoreactant or a complementary immunoreactant.

Chemiluminescent acridinium compounds are each capable of covalent bonding to an antibody or antigen under mild conditions, to become a label having capability of chemiluminescence with high specificity.

The chemiluminescent acridinium compounds include, for example, an acridinium acylchloride having the following formula (1): 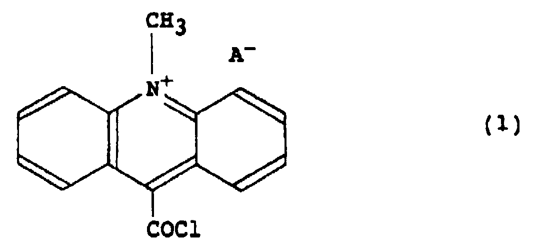 wherein A is an anion, and an acridinium ester having the following formula (2): 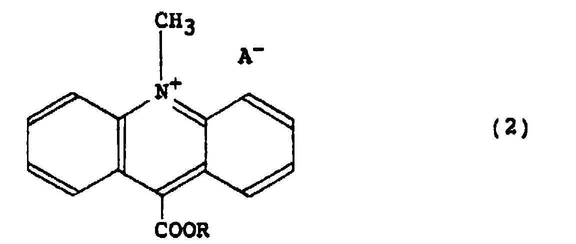 wherein A is an anion, and R is an aliphatic or alicyclic group of from 1 to 12 carbon atoms which may be substituted or unsubstituted or an aromatic group of from 6 to 12 carbon atoms which may be substituted or unsubstituted, which groups may have a reactive group.

In the above formulas (1) and (2), the anion A can be exemplified by anions of tatrafluoroborate perchlorate, halogens such as chlorine and bromine, sulfates such as fluorosulfate, alkyl sulfonates such as ethyl sulfonats, and aryl sulfonate such as phenyl sulfonate. Of the groups suited to R in the formula (2), the aliphatic groups include, for example, alkyl groups such as methyl, etc.; the alicyclic groups include, for example, cycloalkyl groups such as cyclohexyl, etc.; and the aromatic groups include, for example, aryl groups such as phenyl, naphthyl, etc. and aralkyl groups such as benzyl, phenylethyl, and so on. Further, the reactive groups which may be possessed by the above aliphatic, alicyclic or aromatic groups include, for example, amino groups, carbonyl group, and succinimidyl group. Particularly, typical examples of the acridinium esters of the formula (2) include acridinium-I, namely, 4-(2-succinimidyloxycarbonylethyl)phenyl-10-methylacridinium-9-carboxylate fluorosulfate. Besides the above acridinium compounds, the chemiluminescent acridinium compounds described in Japanese Pre-examination Patent Publication (KOKAI) No. 63-57572 (1988) and Japanese Pre-Examination Patent Publication (KOKAI) No. 1-261461 (1989), and the chemiluminescent acridinium esters described, for example, in U.S. Patent No. 4,123,496, U.S. Patent No. 3,352,791, British Patent Nos. 1,316,363 and 1,461,877, and Japanese Pre-examination Patent Publication (KOKAI) No. 62-61969 (1984), can also be used.

The labeling of the immunoreactant or complementary immunoreactant with the above chemiluminescent acridinium compound can be carried out easily, for example, by adding the chemiluminescent acridinium compound to a diluted aqueous alkali solution of an antibody or antigen to react the acridinium compound with the antibody or antigen. The reaction may be performed under mild conditions. For instance, stirring the reaction mixture at a temperature of from 0°C to around room temperature can cause the reaction to proceed sufficiently, giving a labeled immunoreactant or complementary immunoreactant. It is advantageous that the chemiluminescent acridinium compound attached to the antibody or antigen as a marker exhibits an extremely high intensity of luminescence, the intensity being several tens of times that attainable from luminol with aid of peroxidase enhancers, for example. The mechanism of the luminescence of the acridinium compounds has been well known, and it is also advantageous that the luminescence takes place in the presence of hydrogen peroxide without any special catalyst.

### Electrochemiluminescence

In the present invention, the above-described labeled immunoreactant or complementary immunoreactant is immobilized onto the aforesaid electrode by the immunoreaction, or antigen-antibody reaction, and electrochemical reduction of dissolved oxygen in an electrolytic solution is carried out with the electrode as a working electrode (cathode), thereby causing chemiluminescence. Namely, the electrochemical reduction of the dissolved oxygen produces hydrogen peroxide on the working electrode, and the hydrogen peroxide comes into contact with the labeled immunoreactant or complementary immunoreactant (which may hereinafter be referred to simply as "labeled reactant") immobilized on the working electrode, to cause luminescence to take place.

The electrolytic solution for use in the present method should indispensably contain dissolved oxygen, and the amount of the dissolved oxygen should be large enough to generate a sufficient amount of hydrogen peroxide for triggering the luminescence of the labeled reactant (labeled with the chemiluminescent acridinium compound) immobilized on the electrode. Usually, this condition rarely fails to be fulfilled. Where the amount of dissolved oxygen might be insufficient, air or a gas containing oxygen at not less than a certain partial pressure may be introduced into the electrolytic solution prior to measurement, whereby sufficient dissolved-oxygen amount can be ensured.

Generally, as the electrolytic solution, ordinarily, phosphate buffer saline (PBS) is used suitably.

The potential of the working electrode necessary for the luninescence based on electrochemical reduction of dissolved oxygen depends on the characteristics of the electrolytic solution and electrode material used, environmental conditions, and so on. For example, where PBS of pH 7 to 7.4 is used as the electrolytic solution and a platinum electrode is used as the electrode (antigen or antibody electrode) at room temperature, a potential of from -1.5 to -0.2 V vs. Ag/AgCl may be suitable.

Electrodes which can be used as the counter electrode (anode) include, for example, electrodes of metals such as platinum, gold and the like, electrodes of carbon such as graphite, glassy carbon and the like, and oxide electrodes such as ITO electrodes.

The amount of luminescence generated as above is measured, for example, by a photo detector such as photomultiplier tube, etc.

The amount of luminescence is dependent on the amount of the labeled reactant bound to the electrode, and the amount of the bound labeled reactant depends on the concentration of the immunoreactant to be measured in the liquid sample. Therefore, once the amounts of luminescence have been measured for a number of liquid samples containing an immunoreactant in known concentrations and a calibration curve has been prepared, the concentration of the immunoreactant in a given liquid sample can be calculated by measuring the amount of luminescence for the given liquid sample.

The first invention and the second invention will now be individually explained below. For convenience, the following explanations will be made with reference to the case where the immunoreactant present in a liquid sample to be measured is an antigen, and the complementary immunoreactant therefor is a complementary antibody.

### First Invention

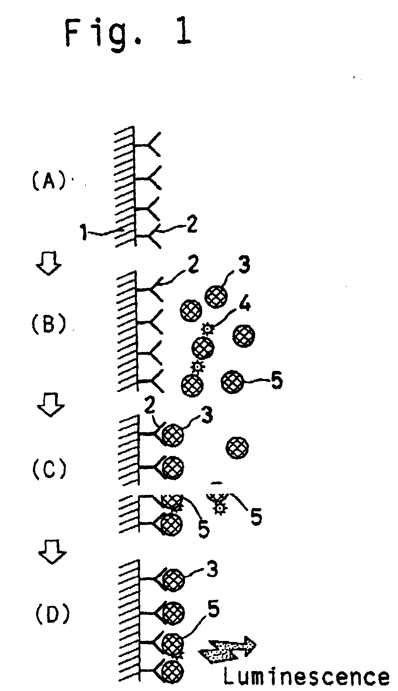

The steps of the measuring method according to the first invention are schematically shown in Figure 1.

First, in step (A) shown in Figure 1, a complementary antibody 2 for an antigen contained in a liquid sample to be assayed is immobilized onto an electrode 1. The electrode 1 with the complementary antibody 2 immobilized thereon will be referred to as "antibody electrode".

Next, in step (b), a labeled antigen 5 which is labeled with a chemiluminescent acridinium compound 4 is added to a liquid sample containing an unlabeled antigen 3 to be measured, and the resulting liquid is brought into contact with the antibody electrode 1.

The chemiluminascent acridinium compound 4 used here as a label, or marker, performs chemiluminescence in the presence of hydrogen peroxide, as described above, the intensity of luminescence being extremely high. In general, the amount of the labeled antigen added to the liquid sample is controlled to be in excess with respect to the amount of the immobilized antibody on the electrode as conventionally carried out in the competitive technique. If the amount of the labeled antigen 5 is too excessively large or too excessively small, however, change in the amount of luminescence due to change in the concentration of unlabeled antigen may be too slight to give satisfactory measurement accuracy.

Further, in step (C), the unlabeled antigen 3 and the labeled antigen 5 in the liquid are competitively bound to the antibody electrode 1 by an immunoreaction, or antigen-antibody reaction, and then excess free unlabeled antigen 3 and labeled antigen 5 are washed away.

The immunoreaction is a competitive reaction of the unlabeled antigen 3 and the labeled antigen 5 with the immobilized antibody 2 on the electrode 1, so that the amount of the labeled antigen 5 bound onto the antibody electrode 1 depends on the concentration of the unlabeled antigen 3 in the liquid sample. Namely, the higher the concentration of the unlabeled antigen 3 to be measured, the smaller the amount of the bound labeled antigen 5; the lower the concentration of the unlabeled antigen 3 to be measured, the larger the amount of the bound labeled antigen 5.

The washing away of the unbound or free unlabeled antigen 3 and labeled antigen 5 after completion of the immunoreaction is carried out by taking the antibody electrode 1 out of the liquid sample and washing the electrode with a cleaning liquid. The cleaning liquid may be any liquid, provided that the liquid does not have bad effect on the labeled antigen 5 bound to the immobilized antibody 2. In general, a phosphate buffer saline (PBS) is used preferably.

Subsequently, in stop (D), electrochemiluminescence is effected by use of the electrode 1. As has been detailed above, the electrochemiluminescence is generated by electrochemical reduction of dissolved oxygen in an electrolytic liquid, with the electrode 1 as a working electrode (cathode). That is to say, the reduction of oxygen causes generation at the working electrode (cathode) of hydrogen peroxide, which triggers the chemiluminescence of the acridinium compound 4 attached to the bound labeled antigen 5 on the electrode.

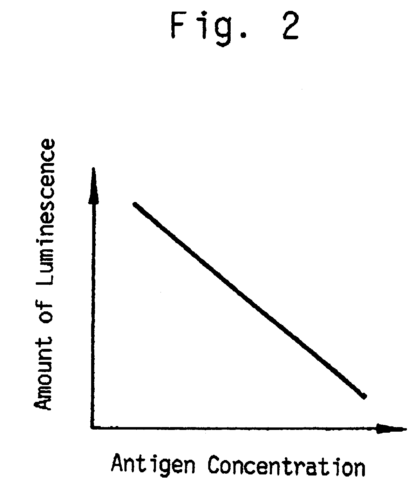

The amount of luminescence varies in proportion to the amount of the bound labeled antigen 5. Therefore, the higher the concentration of the unlabeled antigen 3 in a liquid sample, the smaller the luminescence; the lower the concentration of the unlabeled antigen 3, the larger the luminescence. Accordingly, each calibration curve obtained in the method of the first invention generally has a pattern as shown in Figure 2. The concentration of an antigen in a liquid sample is determined from the measured amount of luminescence, based on the calibration curve.

### Second Invention

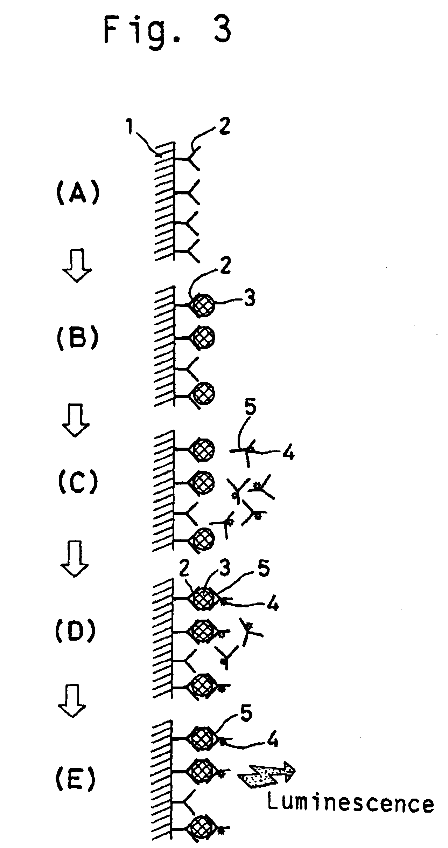

The second invention is substantially the same as the first invention, except that a labeled immunoreactant is bound to an electrode through two immunoreactions (antigen-antibody reactions). The steps of the measuring method of the second invention are schematically illustrated in Figure 3.

First, in step (A) in Figure 3, a complementary antibody 2 for an antigen 3 in a liquid sample to be measured is immobilized onto an electrode 1 to form an antibody electrode, in the same manner as in the first invention.

Next, in step (B), the antibody electrode 1 is immersed in the liquid sample containing the antigen 3 to be measured, and a first immunoreaction is effected. By the first immunoreaction, the antigen 3 contained in the liquid sample is substantially entirely bound to the antibody 2 of the antibody electrode 1.

Subsequently, in steps (C) and (D), an antibody 5 labeled with a chemiluminescent acridinium compound 4 is added to the liquid sample, and a second immunoreaction takes place between the labeled antibody 5 and the bound antigen 3 on the electrode. Thus, in the second invention, the labeled antibody 5 is bound to the electrode 1 through the antigen 3 to be measured. Accordingly, the amount of the labeled antibody 5 bound to the electrode 1 is proportional to the concentration of the antigen 3 in the liquid sample.

Besides, in the second invention, the antibody electrode 1 and the labeled antibody 5 are prepared by use of respective antibodies, which are preferably monoclonal antibodies each capable of binding to a different antigenic determinant. Furthermore, the labeled antibody 5 is preferably added in excess with respect to the amount of the immobilized unlabeled antibody 2 on the electrode 1 so as to react with all of the antigen 3 bound to the immobilized antibody 2 on the electrode 1.

As in the first invention, further, unbound or free labeled antibody 5 is washed away. In step (E), electrochemiluminescence is performed by using as a working electrode (cathode) the electrode with the labeled antibody 5 bound thereto, and the amount of luminescence is measured, from which the concentration of the antigen 3 in the liquid sample is calculated.

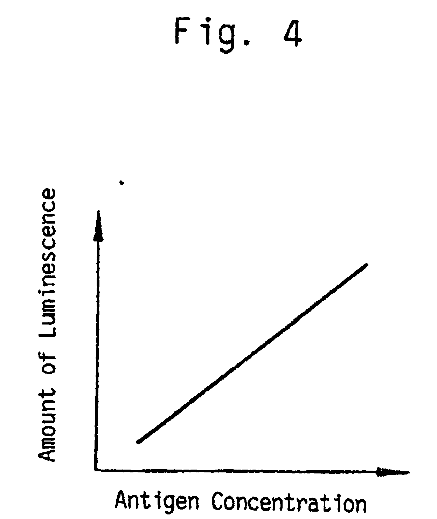

The amount of the bound labeled antibody 5 which generates luminescence is proportional to the concentration of the antigen 3 to be assayed in the liquid sample. Therefore, calibration curves in this method are as shown in Figure 4, representing a pattern reverse to that in the first invention (see Figure 2).

### Liquid Sample

Liquid samples to which the methods of the present invention are applied include, for example, various biological liquids such as blood, lymph, cerebrospinal fluid, urine, sweat, tear, snivel, transcellular fluid, and metabolite liquids, which may be diluted as necessary.

### EXAMPLES

### Immobilization of anti-human-IgG antibody onto electrode

After sufficient washing of an ITO electrode with water, water was removed using acetone. The electrode was then immersed in a 2% acetone solution of gamma-aminopropyltriethoxysilane and left to stand at room temperature for 30 minutes. The thus silane-treated electrode was washed with distilled water several times, and then immersed in a 5% aqueous glutaraldehyde (GA) solution and left to stand at room temperature for 3 hours. After removal of GA, the GA-treated electrode was washed with PBS until the aldehyde odor was eliminated.

The electrode thus treated was immersed in an aqueous solution containing an excess amount of anti-human-IgG (about 10 to 20 µg/ml) and reacted at room temperature for 1 hour. The electrode was then washed several times with PBS, and treated with 1% ethanolamine for about 30 to 60 minutes to block active groups remaining on the surface of the electrode.

### Labeling of human IgG antigen with acridinium compound

Human IgG was dissolved in 0.25 M sodium carbonate buffer (pH 9.0, prepared by mixing 0.25 M of sodium carbonate and sodium hydrogencarbonate), and the solution was diluted with a diluent to a human IgG concentration of 20 mg/ml.

To the diluted solution was added 4-(2-succinimidyloxycarbonylethyl)phenyl-10-methylacridinium-9-carboxylate fluorosulfate as the acridinium compound in an amount of 0.05 g per 1 mg of the human IgG, and permitted to react at room temperature for 3 hours, with gentle stirring.

Free acridinium compound unbound to the antigen was removed by fractionation using a Sephadex G-25 column (tradename, product by Pharmacia) equilibrated with PBS. In the fractionation, PBS was used as an eluting solution.

The rough fraction of human IgG was circulated overnight through an affinity column at 4°C. Then, the acridinium compound adsorbed on the column was washed away with a PBS of pH 7.4. Thereafter, active acridinium-labeled antigen was eluted with a glycine-HCl buffer of pH 2.3. followed by neutralisation with NaOH and dialysis against a PBS of pH 7.4. Thus, an acridinium-labeled antigen was prepared.

### Example 1

To a PBS containing a human IgG to be measured in a known concentration was added the labeled-antigen solution prepared as above (labeled-antigen concentration: 1 µg/ml) in an amount of 1 ml per 1 ml of the PBS, thereby preparing a liquid mixture. In the liquid mixture, the antibody electrode prepared as above was immersed to perform an antigen-antibody reaction for 2 hours, thereby allowing the unlabeled antigen and labeled antigen in the liquid mixture to bind to the immobilized antibody on the electrode. The electrode was then taken out of the liquid mixture, and washed three times with PBS to separate the free unlabeled and labeled antigens.

With the thus treated electrode as a working electrode and a platinum electrode as a counter electrode, electrochemiluminescence was effected by giving a potential of -1.3 V vs. Ag/AgCl to the working electrode for 30 seconds. For this luminescence, a PBS of pH 7.4 was used as an electrolytic solution. The amount of luminescence for the 30-second period was measured and integrated by a photo-counting method.

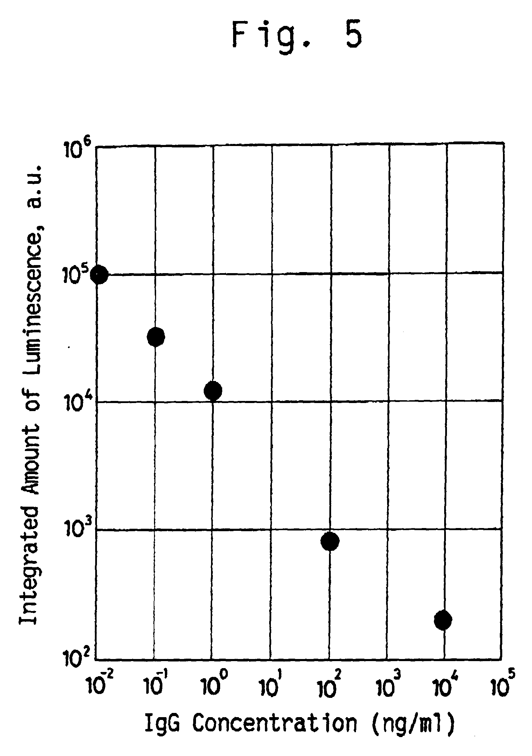

The above measurement was repeated for various PBS's having respectively known antigen concentrations, in order to determine the relationship between antigen concentration and integrated amount of luminescence. The results are represented in the diagram shown in Figure 5.

Further, amount of luminescence was measured for several liquid samples having unknown human IgG concentrations, in the same manner as above, and the antigen concentrations were determined from the diagram (calibration curve) of Figure 5. The measured values were in good agreement with those obtained by a conventional enzyme immunoassay.

### Labeling of anti-human-IgG antibody with acridinium compound

Anti-human-IgG was dissolved in a 0.25 M sodium carbonate buffer (pH 9.0, obtained by mixing 0.25 M sodium carbonate with sodium hydrogencarbonate). The resulting solution was diluted with a diluent to an anti-human-IgG concentration of 20 mg/ml. To the diluted solution thus obtained, 4-(2-succinimidyloxycarbonylethyl)phenyl-10-methylacridinium-9-carboxylate fluorosulfate as the acridinium compound was added in an amount of 0.05 g per 1 mg of the anti-human-IgG, and permitted to react at room temperature for 3 hours, with gentle stirring.

Free acridinium compound unbound to the antibody was removed by fractionation using a Sephadex G-25 column (tradename, product by Pharmacia) equilibrated with PBS. In the fractionation, a PBS was used as an eluting solution.

The rough fraction of anti-human-IgG solution was circulated overnight through an affinity column at 4°C. Then, the acridinium compound adsorbed in the column was washed away with a PBS of pH 7.4. Thereafter, active acridinium-labeled antibody was eluted with a glycine-HCl buffer of pH 2.3, followed by neutralization with NaOH and dialysis against a PBS of pH 7.4. Thus, an antigen labeled with the acridinium compound was prepared.

### Example 2

The ITO antibody electrode prepared as above was immersed in a liquid sample consisting of a PBS containing an antigen to be measured (human IgG) in a known concentration, and an antigen-antibody reaction was permitted to take place for 1 hour, thereby allowing the antigen in the liquid sample to bind to the immobilized antibody on the electrode. The labeled-antibody solution (labeled-antibody concentration: 1 µg/ml) prepared above was added to the liquid sample thus treated in an amount of 1 ml per 1 ml of the liquid sample, to prepare a liquid mixture. In the liquid mixture, the antibody electrode prepared as above was immersed, and an antigen-antibody reaction was permitted to take place for 1 hour, thereby allowing the labeled antibody bind to the antigen immobilized as above. The electrode was then taken out of the liquid mixture, and washed three times with PBS to separate the excess or free labeled antibody therefrom.

With the thus treated electrode as a working electrode and a platinum electrode as a counter electrode, electrochemiluminescence was effected by giving a potential of -1.3 V vs. Ag/AgCl to the working electrode for 30 seconds. For this luminescence, a PBS of pH 7.4 was used as an electrolytic solution. The amount of luminescence for the 30-second period was measured and integrated by a photo-counting method.

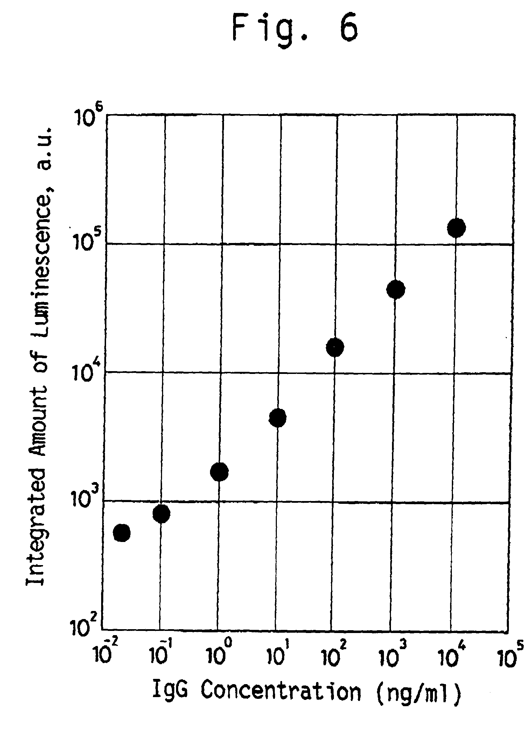

The above measurement was repeated for various PBS's having respectively known antigen concentrations, in order to determine the relationship between antigen concentration and integrated amount of luminescence. The results are shown in the diagram of Figure 6.

Further, amount of luminescence was measured for several liquid samples having unknown human IgG concentrations, in the same manner as above, and the antigen concentrations were determined from the diagram (calibration curve) of Figure 6. The measured values showed good agreement with those obtained by a conventional enzyme immunoassay.

### Example 3

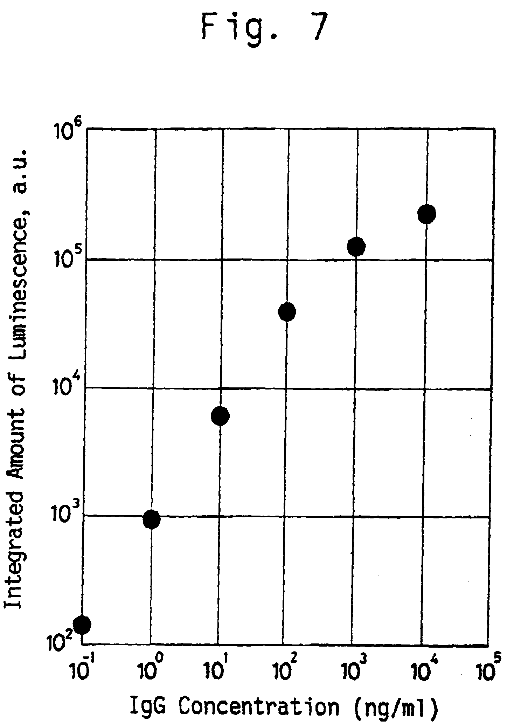

Luminescence was permitted to occur in the same manner as in Example 2, except that a platinum electrode with anti-IgG antibody immobilized thereon was used in place of the antibody immobilized ITO electrode and that a potential of -1.5 V vs. Ag/AgCl was given to the electrode. Measurements of the relationship between antigen concentration and maximum amount of luminescence gave the results as represented by the drawing in Figure 7.

## Claims

1. A method of measuring the concentration of an immunoreactant in a liquid sample, comprising the steps of:
adding to the immunoreactant-containing liquid sample an immunoreactant competitive with the immunoreactant in the liquid sample, said competitive immunoreactant having been previously labeled with a chemiluminescent acridinium compound;
causing an immunoreaction between the unlabeled and labeled immunoreactants with a complementary immunoreactant which is capable of specifically combining with the immunoreactants and is immobilized on an electrode, to allow competitive binding of the unlabeled and labeled immunoreactants onto the electrode to take place;
washing the electrode to remove the excess or unbound unlabeled and labeled immunoreactants; and
subjecting dissolved oxygen in an electrolytic solution to electrochemical reduction using the washed electrode as a working electrode to generate luminescence; and
measuring the amount of luminescence.

2. The method according to claim 1, wherein the labeled immunoreactant is added to the livid sample in an excessive amount with respect to the immobilized complementary immunoreactant on the electrode.

3. The method according to claim 1, wherein the chemiluminescent acridinium compound is an acridinium acylchloride having the following formula (1): 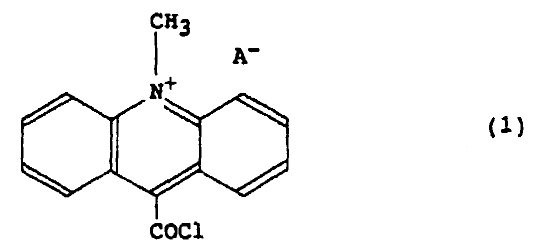 wherein A is an anion, or an acridinium ester having the following formula (2): 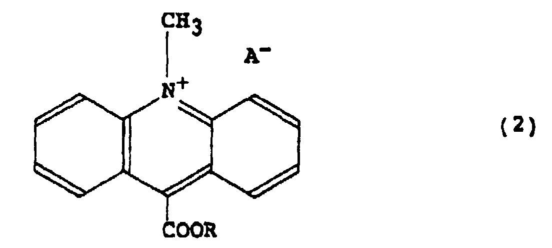 wherein A is an anion, and R is an aliphatic or alicyclic group of from 1 to 12 carbon atoms which may be substituted or unsubstituted or an aromatic group of from 6 to 12 carbon atoms which may be substituted or unsubstituted, which groups may have a reactive group.

4. A method of measuring the concentration of an immunoreactant in a liquid sample, comprising the steps of:
causing an immunoreaction between the immunoreactant with a complementary immunoreactant which is capable of specifically combining with the immunoreactant to be measured and is immobilized on an electrode in the liquid sample, to allow binding of the immunoreactant in the liquid sample onto the electrode to take place;
adding an excess of a complementary immunoreactant labeled with a chemiluminescent acridinium compound to the liquid thus obtained, to allow the bound immunoreactants to react with the labeled complementary immunoreactant;
washing the electrode to remove the excess or unbound labeled complementary immunoreactant; and
subjecting dissolved oxygen in an electrolytic solution to electrochemical reduction using the washed electrode as a working electrode to generate luminescence; and
measuring the amount of luminescence.

5. The method according to claim 4, wherein the chemiluminescent acridinium compound is an acridinium acylchloride having the following formula (1): 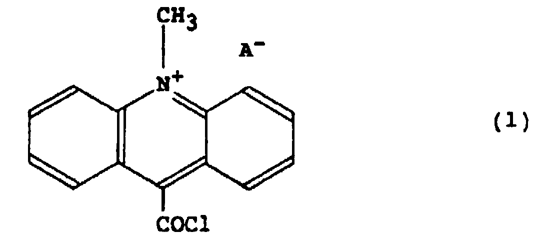 wherein A is an anion, or an acridinium ester having the following formula (2): 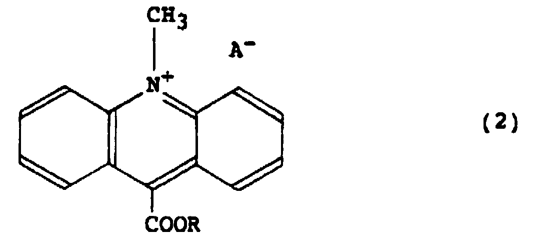 wherein A is an anion, end R is an aliphatic or alicyclic group of from 1 to 12 carbon atoms which may be substituted or unsubstituted or an aromatic group of from 6 to 12 carbon atoms which may be substituted or unsubstituted, which groups may have a reactive group.

## Patentansprüche

1. Verfahren zum Messen der Konzentration eines Immunoreaktanten in einer flüssigen Probe, umfassend die Schritte:
Zugeben eines mit dem Immunoreaktanten in der flüssigen Probe kompetitiven Immunoreaktanten zu der flüssigen, Immunoreaktanten-enthaltenden Probe, wobei der kompetitive Immunoreaktant zuvor mit einer chemilumineszenten Acridiniumverbindung markiert worden ist,
Herbeiführen einer Immunreaktion zwischen den unmarkierten und markierten Immunoreaktanten mit einem komplementären Immunoreaktant, der zur spezifischen Bindung mit den Immunoreaktanten befähigt ist und der auf einer Elektrode immobilisiert ist, um das Stattfinden einer kompetitiven Bindung der unmarkierten und markierten Immunoreaktanten auf der Elektrode zu ermöglichen,
Waschen der Elektrode, um den Überschuß an Immunoreaktanten oder ungebundene, unmarkierte und markierte Immunoreaktanten zu entfernen, und
Unterwerfen von gelöstem Sauerstoff in einer elektrolytischen Lösung einer elektrochemischen Reduktion unter Verwendung der gewaschenen Elektrode als Arbeitselektrode zur Erzeugung von Lumineszenz, und
Messen der Lumineszenzmenge.

2. Verfahren nach Anspruch 1, worin der markierte Immunoreaktant zur flüssigen Probe in einer überschüssigen Menge bezüglich des immobilisierten komplementären Immunreaktanten auf der Elektrode zugegeben wird.

3. Verfahren nach Anspruch 1, wobei die chemilumineszente Acridiniumverbindung ein Acridiniumacylchlorid mit der folgenden Formel (1) ist 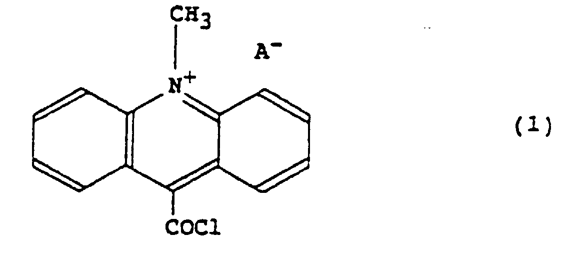 wobei A ein Anion ist, oder ein Acridiniumester mit der folgenden Formel (2) ist 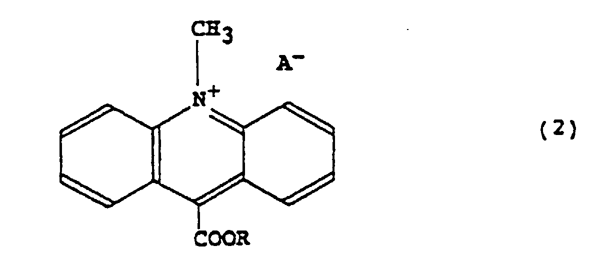 wobei A ein Anion ist und R ein aliphatischer oder alicyclischer Rest mit 1 bis 12 Kohlenstoffatomen, die substituiert oder unsubstituiert sein können, oder ein aromatischer Rest mit 6 bis 12 Kohlenstoffatomen, die substituiert oder unsubstituiert sein können, ist, wobei die Reste eine reaktive Gruppe aufweisen.

4. Verfahren zum Messen der Konzentration eines Immunoreaktanten in einer flüssigen Probe, umfassend die Schritte
Herbeiführen einer Immunreaktion zwischen dem Immunoreaktanten mit einem komplementären Immunoreaktanten, der zur spezifischen Bindung mit dem zu messenden Immunoreaktanten befähigt ist und der auf einer Elektrode immobilisiert ist, in der flüssigen Probe, um das Stattfinden einer Bindung des Immunoreaktanten in der flüssigen Probe auf der Elektrode zu ermöglichen,
Zugeben eines Überschusses des mit einer chemilumineszenten Acridiniumverbindung markierten, komplementären Immunoreaktanten zu der so erhaltenen Flüssigkeit, um eine Reaktion des gebundenen Immunoreaktanten mit dem markierten komplementären Immunoreaktanten zu ermöglichen,
Waschen der Elektrode, um den Überschuß an Immunoreaktanten oder ungebundenen markierten komplementären Immunoreaktanten zu entfernen, und
Unterwerfen von gelöstem Sauerstoff in einer elektrolytischen Lösung einer elektrochemischen Reduktion unter Verwendung der gewaschenen Elektrode als Arbeitselektrode zur Erzeugung von Lumineszenz, und
Messen der Lumineszenzmenge.

5. Verfahren nach Anspruch 4, wobei die chemilumineszente Acridiniumverbindung ein Acridiniumacylchlorid mit der folgenden Formel (1) ist 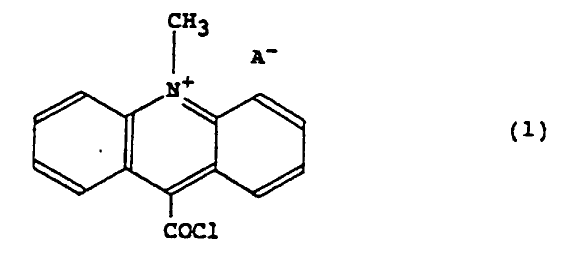 wobei A ein Anion ist, oder ein Acridiniumester mit der folgenden Formel (2) ist 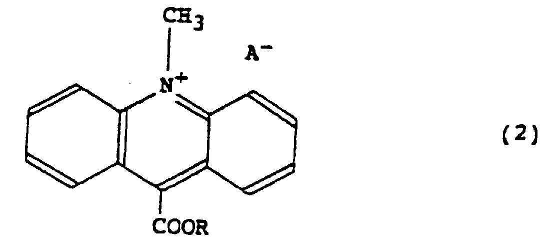 wobei A ein Anion ist und R ein aliphatischer oder alicyclischer Rest mit 1 bis 12 Kohlenstoffatomen, die substituiert oder unsubstituiert sein können, oder ein aromatischer Rest mit 6 bis 12 Kohlenstoffatomen, die substituiert oder unsubstituiert sein können, ist, wobei die Reste eine reaktive Gruppe aufweisen.

## Revendications

1. Procédé pour mesurer la concentration d'un immunoréactif dans un échantillon de liquide, comprenant les étapes consistant à :
ajouter, à l'échantillon de liquide contenant un immunoréactif, un immunoréactif compétitif de l'immunoréactif dans l'échantillon de liquide, ledit immunoréactif compétitif ayant été marqué au préalable avec un composé d'acridinium chimioluminescent ;
provoquer une réaction immunologique entre les immunoréactifs non marqué et marqué et un immunoréactif complémentaire qui est capable de se combiner spécifiquement avec les immunoréactifs et qui est immobilisé sur une électrode, pour permettre qu'ait lieu la fixation compétitive des immunoréactifs non marqué et marqué sur l'électrode ;
laver l'électrode pour enlever les immunoréactifs non marqué et marqué en excès ou non fixés ; et
soumettre de l'oxygène dissous dans une solution électrolytique à une réduction électrochimique utilisant l'électrode lavée en tant qu'électrode de travail pour engendrer une luminescence ; et
mesurer la quantité de luminescence.

2. Procédé selon la revendication 1, dans lequel l'immunoréactif marqué est ajouté à l'échantillon de liquide en une quantité excessive par rapport à l'immunoréactif complémentaire immobilisé sur l'électrode.

3. Procédé selon la revendication 1, dans lequel le composé d'acridinium chimioluminescent est un acylchlorure d'acridinium ayant la formule (1) suivante : 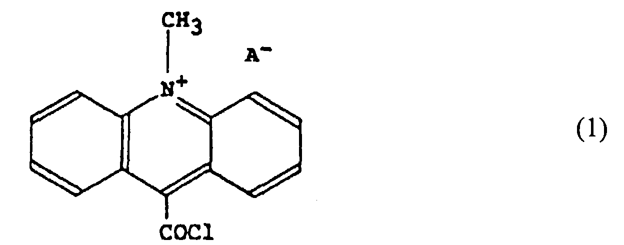 dans laquelle A est un anion, ou un ester d'acridinium ayant la formule (2) suivante : 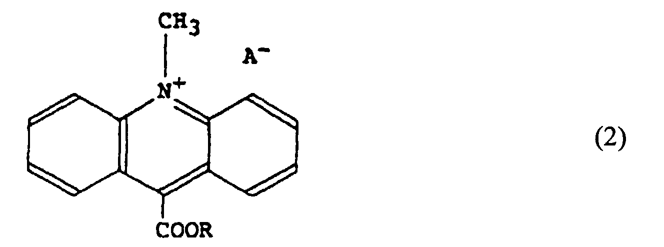 dans laquelle A est un anion, et R est un groupe aliphatique ou alicyclique éventuellement substitué contenant de 1 à 12 atomes de carbone, ou un groupe aromatique éventuellement substitué contenant de 6 à 12 atomes de carbone, ces groupes pouvant porter un groupe réactif.

4. Procédé pour mesurer la concentration d'un immunoréactif dans un échantillon de liquide, comprenant les étapes consistant à :
provoquer une réaction immunologique entre l'immunoréactif et un immunoréactif complémentaire qui est capable de se combiner spécifiquement avec l'immunoréactif devant être mesuré et qui est immobilisé sur une électrode dans l'échantillon de liquide, pour permettre qu'ait lieu la fixation de l'immunoréactif dans l'échantillon de liquide sur l'électrode ;
ajouter au liquide ainsi obtenu un excès d'un immunoréactif complémentaire marqué avec un composé d'acridinium chimioluminescent, pour permettre aux immunoréactifs fixés de réagir avec l'immunoréactif complémentaire marqué ;
laver l'électrode pour enlever l'immunoréactif complémentaire marqué en excès ou non fixé ; et
soumettre de l'oxygène dissous dans une solution électrolytique à une réduction électrochimique utilisant l'électrode lavée en tant qu'électrode de travail pour engendrer une luminescence ; et
mesurer la quantité de luminescence.

5. Procédé selon la revendication 4, dans lequel le composé d'acridinium chimioluminescent est un acylchlorure d'acridinium ayant la formule suivante (1) : 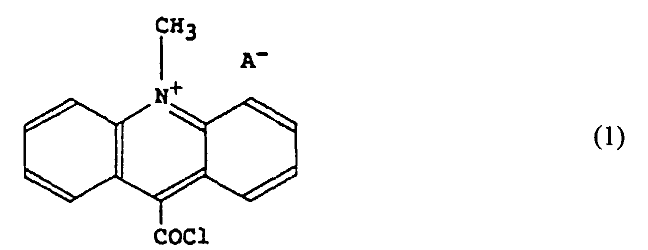 dans laquelle A est un anion, ou un ester d'acridinium ayant la formule (2) suivante : 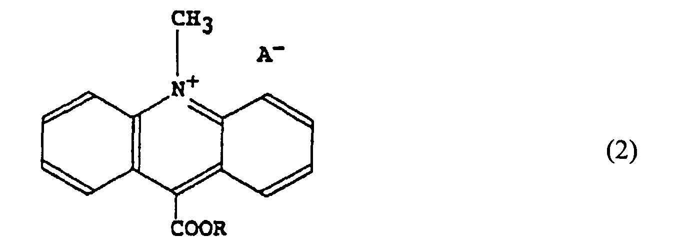 dans laquelle A est un anion, et R est un groupe aliphatique ou alicyclique éventuellement substitué contenant de 1 à 12 atomes de carbone, ou un groupe aromatique éventuellement substitué contenant de 6 à 12 atomes de carbone, ces groupes pouvant porter un groupe réactif.
